# EUROPEAN PATENT APPLICATION

(11) **EP 3 560 488 A1**
(43) Date of publication of application: **30.10.2019**
(21) Application number: 18382276.6
(22) Date of filing: 24.04.2018
(51) Int. Cl.: A61K 9/20, A61K 36/16, A61K 36/18, A61K 36/185, A61K 36/48

(54) **COMPOSITIONS FOR TREATING FEMALE SEXUAL DYSFUNCTION**

(71) Applicant: Procare Health Iberia, S.L., 46450 Benifaió (ES)
(72) Inventor: LOSA DOMÍNGUEZ, Fernando, 08022 BARCELONA (ES); PALACIOS, Santiago, 28009 MADRID (ES); GASLAIN, Yann, 00860 CASTELLDEFELS (ES)
(74) Representative: ZBM Patents - Zea, Barlocci & Markvardsen

(57) **Abstract**

The present invention provides a combination comprising extracts of *Trigonella foenum graecum, Turnera diffusa,* and *Ginkgo biloba* at a weight ratio from 6:1:1 to 24:6:1, as well as combinations further comprising a *Tribulus terrestris* extract, vitamins and/or selenium. The invention also provides pharmaceutical compositions comprising the combinations above described together with one or more appropriate pharmaceutically acceptable excipients and/or carriers, and combinations or pharmaceutical composition for the treatment and/or prevention of female sexual dysfunction, in particular when it is caused by a hormonal imbalance condition such as pregnancy, postpartum, breastfeeding, hormonal contraception, perimenopause, and postmenopause.

## Description

### Technical Field

The present invention belongs to the field of compositions based on plant extracts. In particular, the invention relates to compositions for the treatment of female sexual dysfunction. The composition of the invention is particularly useful for treating women with hormonal imbalance conditions such as perimenopause or postmenopause.

### Background Art

Low sexual desire affects more than 20% of women. A survey among American and European women (Women's International Study of Health and Sexuality, WISHeS) that evaluated the prevalence of hypoactive sexual desire concluded that among women aged 20-49, in their reproductive age, the prevalence of reduced desire causing discomfort and concern was 7%. In women aged 50-70, the prevalence was 9% in women with natural menopause, and 12% in women subject to surgical menopause.

After interviewing 750 women, the most common sexual issues described in the Spanish population were lack of sexual interest (36.0%), impossibility of having an orgasm (27.8%), and non-satisfactory sexual relationships (25.1%).

Women with low sexual desire may have problems when starting or having stable sexual relationships, and they may feel unsatisfied and experience marital disorders. Studies have demonstrated that women with low desire, low excitement, or sexual pain are clearly associated with negative feelings regarding their physical and emotional satisfaction, as well as their happiness. In addition, women suffering from those problems tend to experience much more negative emotions and psychological states than women with a normal sexual activity.

Biological, psychological, and interpersonal factors play a part in sexual dysfunction. The hormones impacting female sexuality (estrogens and androgens) play a crucial role in sexual function. Various studies have demonstrated that reduced testosterone levels are directly related to a decrease sexual activity in postmenopausal women.

Even though testosterone's effectiveness when treating female sexual interest disorders, both in surgical menopause women and in natural menopause women, has already been demonstrated, there are some doubts regarding its long-term safety (c.f. Davis S.R. et al., "Efficacy and safety of testosterone in the management of hypoactive sexual desire disorder in postmenopausal women", 2012, J Sex Med, vol. 9(4), pp. 1134-48). This led the US Food and Drug Administration not to authorize the marketing of testosterone patches, paving the way for new strategies to fight this disorder. One of them is flibanserin, a 5HT1ₐ agonist and a 5HT2ₐ antagonist, which has been demonstrated to increase sexual desire, reduce sexual-dysfunction-associated discomfort, and improve sexual activity in premenopausal women with low sexual desire, with a good tolerability, which translated into flibanserin being approved in the US (c.f. Simon J.A. et al., "Efficacy and safety of flibanserin in postmenopausal women with hypoactive sexual desire disorder: results of the SNOWDROP trial" 2014, Menopause, vol. 21(6), pp. 633-40). However, this central effect drug also has some side effects, which has led to some controversy about its use (c.f. Aftab A. et al., "Flibanserin and its discontents" 2017, Arch Womens Ment Health, vol. 20(2), pp.243-247).

One interesting alternative for treating female sexual disorders are compositions based on natural plant extracts. However, plant extracts have usually presented several disadvantages, such as inconsistent results, low efficacy and, importantly, they generally do not improve all domains of sexual function (i.e. desire, excitement, orgasm, and painful sexual activity) providing only a partial relief to the subjects suffering from sexual dysfunction.

Therefore, there is still a need for safe and effective treatments based on plant extracts to improve sexual function in women, in particular treatments that impact on all the different domains of female sexual function.

### Summary of Invention

The present inventors have developed a novel combination based on natural products for combating sexual dysfunction in women. The combination of the invention comprises a mixture of extracts from *Trigonella foenum graecum, Turnera ditfusa,* and *Ginkgo biloba* plants at specific concentration ratios.

Although it was known that some of the abovementioned plant extracts have a certain aphrodisiac effect, their combined used at the specific ratios herein provided has not been disclosed before. Seeking natural products that could help combating female sexual dysfunction, the present inventors surprisingly found through extensive experimentation that the combination of *Trigonella foenum graecum, Turnera diffuse* and *Ginkgo biloba* extracts at particular concentration ratios has an unexpected effect on every domain of female sexual function, an effect which is not observed when any of the three extracts is not included in the combination, or even when the combination is performed at different ratios. This effect is higher than the sum of effects of the combination of *Trigonella foenum graecum, Turnera diffusa* and *Ginkgo biloba* extracts, separately. Therefore, the specific combination of plant extracts herein disclosed has a synergic effect on the treatment or prevention of female sexual dysfunction.

As shown in the examples below, the inventors found that the administration of this particular combination elevated circulating testosterone free levels and reduced sex-hormone binding globulin levels in women. After 9 weeks of treatment, the Female Sexual Function Index (FSFI) of treated women showed a significant improvement in the domains of desire, excitement, lubrication, orgasm, and sexual satisfaction. Indeed, the global analysis of all FSFI domains demonstrated an improvement of more than four points after only two months of treatment.

Therefore, in a first aspect, the invention provides a combination comprising extracts of *Trigonella foenum graecum, Turnera ditfusa,* and *Ginkgo biloba* at a weight ratio (weight/weight/weight) from 6:1:1 to 24:6:1.

The combination of the invention presents several advantages over the prior art. First of all, it impacts on all the sexual function domains, including physical (e.g. vaginal lubrication) and psychological (e.g. desire) domains, which provides an important improvement in the whole sexual experience of treated women. Secondly, the composition is based on safe natural products known to be well tolerated and secure for human consumption. The extracts of the composition are easy to obtain and they are inexpensive. Furthermore, since the hormonal changes elicited by the composition of the invention are modest and within physiological ranges, the risk of producing undesired effects after long-term administration are significantly low.

A pharmaceutical composition comprising the combination of the inventions is a safe and efficient therapeutic alternative to testosterone patches or 5HT1ₐ/5HT2 antagonist and avoids the drawbacks related to their long-term administration.

Thus, in a second aspect, the present invention provides a pharmaceutical composition comprising an effective amount of the combination as defined above in the first aspect, together with one or more appropriate pharmaceutically acceptable excipients and/or carriers.

Alterations in the sexual function of women are a widespread problem with strong social and psychological consequences; therefore, the composition of the invention constitutes an important advancement in the field of sexual dysfunction treatment.

In a third aspect, the present invention provides the combination as defined in the first aspect or the pharmaceutical composition defined in the second aspect for use as a medicament.

### Brief Description of Drawings

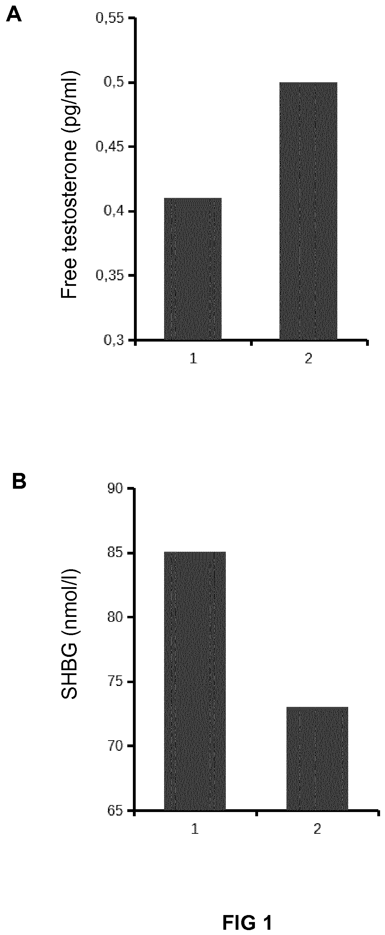

Fig. 1 shows (a) free testosterone and (b) SHBG levels in serum of women before and after 2 months of treatment with the composition of the invention. Column 1 represents women before treatment and column 2 represents women after 2 months of treatment.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

As used herein, "extracts" refer to concentrated preparations of various parts of plants obtained by isolating the active constituents, such as saponines, from the plant by suitable means. Depending of the physico-chemical features of these active ingredients, suitable means for their isolation can be used, for example, aqueous solutions, organic solvents, microwave or supercritical fluids extraction. Plant extracts contain not only one but multiple constituents, many of them biologically active. Often, the beneficial effect is derived from the combination of many of these active compounds, even though in some cases there is one particular compound that is mainly responsible for most of the activity.

The term "weight ratio" refers to the relation of weights of the extracts indicated to treat and/or prevent female sexual dysfunction. For instance, a weight ratio (weight/weight/weight) of 12:3:1 between the *Trigonella foenum graecum, Turnera diffusa,* and *Ginkgo biloba* extracts refers to 12 units of *Trigonella foenum graecum* extract and 3 units of *Turnera diffusa* extract for every 1 unit of *Ginkgo biloba* extract. In the present invention the extracts are dry extracts, therefore the weight is the dry weight.

As used herein, "% by weight" or "% w/w" of a component refers to the amount of the single component relative to the total weight of the composition or, if specifically mentioned, of other component. In the present invention the weight of an extract refers to the dry extract weight or mass.

As used herein, the term "phytosomes" refers to a complex comprising a natural active ingredient or a natural composition, such as a plant extract, and a phospholipid, such as lecithin. The term "liposome" refers to a spherical vesicle having at least one lipid bilayer. The liposome can be used as a vehicle for administration of nutrients and pharmaceutical drugs. "Niosome" refers to a non-ionic surfactant-based vesicle. Niosomes are formed mostly by a non-ionic surfactant and cholesterol.

An "effective amount" of the combination refers to the amount of active ingredients which provide a therapeutic effect after its application or any desired effect.

The term "pharmaceutically acceptable" refers to that excipients or carriers suitable for use in the pharmaceutical technology for preparing compositions with medical use, in particular, for preparing compositions for oral administration.

As used herein, the term "extract ratio" refers to the quantity of dry plant material required to produce a stated quantity of plant extract. For example, an extract ratio of 5:1 means that 5 kg of dry plant material is used to produce 1 kg of plant extract.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the combination of the invention can be presented in any dosage form, for example, solid or liquid, and can be administered by any suitable route, for example, oral, parenteral, for which they will include the pharmaceutically acceptable excipients necessary for the formulation of the desired dosage form. The pharmaceutical composition of the invention is preferably solid and administered by oral route.

The term "female sexual dysfunction" refers to a heterogeneous group of disorders that are typically characterized by a clinically significant disturbance in a female's ability to respond sexually or to experience sexual pleasure. This group includes, for example, Sexual Desire Disorders (such as Hypoactive Sexual Desire Disorder [HSDD] and Sex Aversion Disorder), Female Anorgasmia, Female Sexual Excitement Disorder, and Pain-Associated Sexual Disorders, among others. The compositions of the invention are particularly useful for the treatment of female sexual dysfunction caused by a hormonal imbalance condition.

As used herein "hormonal imbalance condition" refers to situations in which the normal levels of hormones, particularly sexual hormones such as estradiol and testosterone, are altered. In women this can occur, for instance, during pregnancy, lactation, perimenopause, postmenopause and hormonal contraception treatments. Said changes in the level of sexual hormones commonly lead to one or more of the female sexual disorders above mentioned.

As used herein, "hormonal contraception" refers to birth control methods that act on the endocrine system of women.

The term "perimenopause" refers to the stage in a woman's life that begins when the ovaries start to decline in function, and continues until menopause occurs. "Menopause" is the total cessation of menstrual flow for one calendar year. The term "postmenopause" refers to the stage that begins when menopause occurs. The compositions of the invention are particularly useful in the treatment of sexual dysfunction in perimenopausal and postmenopausal women.

The term "dosage unit" refers to a pharmaceutical entity that comprises a pharmacologically active ingredient and pharmaceutical acceptable excipients or carriers and which is actually to be administered to, or to be taken by, a human. The term "pharmaceutical dosage unit" also encompasses non-reusable packaging as well, especially when pharmaceutical composition is individually packaged. Further, the "pharmaceutical dosage unit" of the invention can be obtained from a larger dosage unit prepared in advanced. These larger dosage units are known as "multidose dosage units".

The term "pharmaceutically acceptable" refers to that excipients or carriers suitable for use in the pharmaceutical technology for preparing compositions with medical use.

As indicated above, in a first aspect the invention provides a combination comprising extracts of *Trigonella foenum graecum, Turnera diffusa,* and *Ginkgo biloba* at a weight ratio (weight/weight/weight) from 6:1:1 to 24:6:1. More particularly, the weight ratio is from 9:2:1 to 18:4:1.

In a more particular embodiment of the first aspect, the weight ratio (weight/weight/weight) between the *Trigonella foenum graecum, Turnera diffuse,* and *Ginkgo biloba* extracts is 12:3:1. This means that there are 12 weight units of *Trigonella foenum graecum* extract and 3 weight units of *Turnera diffusa* extract for every weight unit of *Ginkgo biloba* extract.

In a particular embodiment, optionally in combination with any embodiments provided above or below, the composition according to the first aspect further comprises *Tribulus terrestris* extract. Therefore, it is a composition comprising extracts from *Trigonella foenum graecum, Turnera diffuse, Ginkgo biloba,* and *Tribulus terrestris.*

As shown in the examples below, the inventors surprisingly found that the addition of *Tribulus terrestris* extracts to the combination of the invention further strengthens its sexual function booster effect. Importantly, women suffering from sexual dysfunction administered with a composition comprising the four plant extracts showed substantial improvements in all the domains of sexual function as measured by the FSFI.

In a more particular embodiment, the weight ratio (weight/weight/weight/weight) between *Trigonella foenum graecum, Turnera diffusa, Ginkgo biloba* and *Tribulus terrestris* extracts is from 6:1:1:0.2 to 24:6:1:2. More particularly, the weight ratio is from 9:2:1:0.4 to 18:4:1:1.4.

In an even more particular embodiment, the weight ratio (weight/weight/weight/weight) between *Trigonella foenum graecum, Turnera diffusa, Ginkgo biloba* and *Tribulus terrestris* extracts is 12:3:1:0.8. This means that there are 12 weight units of *Trigonella foenum graecum* extract, 3 weight units of *Turnera diffusa* extract, and 0.8 weight units of *Tribulus terrestris* extract for every 1 weight unit of *Ginkgo biloba* extract.

In a particular embodiment of the first aspect, optionally in combination with any embodiments provided above or below, the combination comprises:
- *Trigonella foenum graecum* in an amount from 25 to 65 % w/w,
- *Turnera diffusa* in an amount from 5 to 20 % w/w,
- *Ginkgo biloba* in an amount from 2 to 6 % w/w, and
- *Tribulus terrestris* in an amount from 1 to 5 % w/w.

In a more particular embodiment of the first aspect, the combination comprises:
- *Trigonella foenum graecum* in an amount of 44 % w/w,
- *Turnera diffusa* in an amount of 11 % w/w,
- *Ginkgo biloba* in an amount of 3.7 % w/w, and
- *Tribulus terrestris* in an amount of 2.9 % w/w.

In a particular embodiment, optionally in combination with any embodiments provided above or below, the extracts are dry extracts.

In a particular embodiment, the *Trigonella foenum graecum* extract is a dry seed extract. In a more particular embodiment, it is a seed extract comprising saponins in an amount from 25 to 75 % w/w of dry extract mass. Even more particularly, it is a seed extract containing saponins in an amount of 50 % w/w of dry extract mass. In a particular embodiment, the extract ratio of the *Trigonella foenum graecum* extract is from 4:1 to 8:1. In a more particular embodiment, the extract ratio of the *Trigonella foenum graecum* extract is 6:1. In particular, the extract is obtainable by ethanol extraction.

In a particular embodiment, the *Turnera diffusa* extract is a dry leaf extract. In a particular embodiment, the extract ratio of the *Turnera diffusa* extract is from 2:1 to 6:1. In a more particular embodiment, the extract ratio of the *Turnera diffusa* extract is 4:1. In particular, the extract is obtainable using water as solvent. More particularly, the *Turnera diffusa* extract is the extract of CAS registry number 84696-52-6.

In a particular embodiment, the *Ginkgo biloba* extract is a dry leaf extract. In a more particular embodiment, the *Ginkgo biloba* extract comprises ginkgoflavonoids in an amount from 10 to 40 % w/w of dry extract mass. Even more particularly, the ginkgoflavonoids are in an amount of 25 % w/w of dry extract mass. In a particular embodiment, the extract ratio of the *Ginkgo biloba* extract is from 20:1 to 80:1. In a more particular embodiment, the extract ratio of the *Ginkgo biloba* extract is from 35:1 to 67:1. In particular, the extract is obtainable by acetone and water extraction. In particular, the *Ginkgo biloba* extract comprises ginkgoflavonglucosides in an amount from 6 to 10 % w/w, ginkgoterpenes in an amount from 2 to 6 % w/w, bilobalide in an amount from 1 to 3 % w/w, and ginkgolides in an amount from 1 to 4 % w/w. More particularly, the *Ginkgo biloba* extract comprises ginkgoflavonglucosides in an amount of 7.7 % w/w, ginkgoterpenes in an amount of 3.8 % w/w, bilobalide in an amount from 1.4 % w/w, and ginkgolides in an amount from 2.5 % w/w.

In a particular embodiment, the *Tribulus terrestris* extract is a fruit extract. In a more particular embodiment, it is an extract that comprises saponins in an amount from 70 to 98 % w/w of dry extract mass. More particularly, it comprises saponins in an amount of 90 % w/w of dry extract mass. In a particular embodiment, the extract ratio of the *Tribulus terrestris* extract is from 10:1 to 25:1. In a more particular embodiment, the extract ratio of the *Tribulus terrestris* extract is from 15:1 to 20:1. In particular, the extract is obtainable by ethanol and water extraction. More particularly, the *Tribulus terrestris* extract is the extract of CAS registry number 90131-68-3.

In a particular embodiment of the first aspect, optionally in combination with any embodiments provided above or below, one or more of the extracts are encapsulated into phytosomes, liposomes or niosomes. This embodiment is meant to encompass extracts that are individually encapsulated or two, three or four extracts encapsulated altogether. In a particular embodiment, the *Ginkgo biloba* extract is encapsulated into phytosomes, liposomes or niosomes. The inventors surprisingly found that the efficacy of the combination of the invention was greatly enhanced when the *Ginkgo biloba* extract was encapsulated into phytosomes, a molecular form with enhanced bioavailability. Thus, in a particular embodiment, the *Ginkgo biloba* extract is encapsulated into phytosomes. In a particular embodiment, the phytosomes comprise lecithin. In a more particular embodiment, the phytosomes comprise *Ginkgo biloba* extract in an amount from 20 to 40 % w/w and lecithin in an amount from 60 to 80 % w/w. More particularly, in the phytosomes the *Ginkgo biloba* extract is in an amount of 33 % w/w and lecithin is in an amount of 67 % w/w. In a particular embodiment, the particle size of the phytosomes comprising the *Ginkgo biloba* extract is from 75 to 300 µm.

Vitamins are essential nutrients that play important roles in most of the functions of organism, including the sexual function. The inventors discovered that when vitamins were combined with the four extracts above mentioned, the results obtained in the treatment of sexual dysfunction were highly improved.

Surprisingly, the inventors also found that selenium, an antioxidant known to improve microcirculation, enhanced even further the effects of the combination of the invention.

In a particular embodiment of the first aspect, optionally in combination with any embodiments provided above or below, the combination further comprises vitamins and/or selenium. In a more particular embodiment, the vitamins are selected from the group consisting of vitamin B1, vitamin B2, vitamin B3, vitamin B6, and combinations thereof. In a particular embodiment, the combination comprises vitamin B1, vitamin B2, vitamin B3, vitamin B6 and selenium.

In a particular embodiment of the first aspect, optionally in combination with any embodiments provided above or below, the combination further comprises:
- vitamin B1 in an amount from 0.1 % to 0.4 % w/w,
- vitamin B2 in an amount from 0.2 % to 0.5 % w/w,
- vitamin B3 in an amount from 1 % to 5 % w/w,
- vitamin B6 in an amount from 0.1 % to 0.4 % w/w, and
- selenium in an amount from 0.001 % to 0.007 % w/w.

In a more particular embodiment of the first aspect, the combination further comprises:
- vitamin B1 in an amount of 0.16 % w/w,
- vitamin B2 in an amount of 0.26 % w/w,
- vitamin B3 in an amount of 2.35 % w/w,
- vitamin B6 in an amount of 0.15 % w/w, and
- selenium in an amount of 0.0037 % w/w.

In a particular embodiment of the first aspect, the combination comprises *Trigonella foenum graecum* extracts, *Turnera diffusa* extracts, *Ginkgo biloba* extracts, *Tribulus terrestris* extracts, vitamin B1, vitamin B2, vitamin B3, vitamin B6 and selenium. In a more particular embodiment of the first aspect, the composition consists of *Trigonella foenum graecum* extracts, *Turnera diffusa* extracts, *Ginkgo biloba* extracts, *Tribulus terrestris* extracts, vitamin B1, vitamin B2, vitamin B3, vitamin B6 and selenium.

As detailed above, in a second aspect the invention provides a pharmaceutical composition comprising an effective amount of the combination as defined above in the first aspect, together with one or more appropriate pharmaceutically acceptable excipients and/or carriers.

All the embodiments of the combination of the invention also apply to the pharmaceutical composition provided in the second aspect.

As above indicated, in a third aspect the invention provides the combination as defined in the first aspect or the pharmaceutical composition as defined in the second aspect for use as a medicament.

In a particular embodiment, the combination as defined in the first aspect or the pharmaceutical composition defined in the second aspect is for use in the treatment and/or prevention of female sexual dysfunction. This embodiment can also be formulated as the use of the combination or the pharmaceutical composition as defined above for the manufacture of a medicament, for the treatment and/or prevention of female sexual dysfunction. This embodiment can also be formulated as a method of treating women in need thereof with a combination as defined in the first aspect or a pharmaceutical composition as defined in the second aspect above, in particular for treating and/or preventing female sexual dysfunction.

In a particular embodiment, optionally in combination with any embodiments provided above or below, the female is human.

In a more particular embodiment of the third aspect, the combination as defined in the first aspect or the pharmaceutical composition as defined in the second aspect is for use in the treatment and/or prevention of female sexual dysfunction caused by a hormonal imbalance condition.

There are various conditions that alter the normal levels of sexual hormones in women, and the composition of the invention is effective in the treatment of sexual dysfunction in women under any of these conditions. Thus, in a particular embodiment of the third aspect, the hormonal imbalance condition is selected from the group consisting of pregnancy, postpartum, breastfeeding (lactation), hormonal contraception, perimenopause and postmenopause. All these conditions produce alterations in the normal level of hormones, particularly in sexual hormones such as testosterone and estradiol.

At the onset of menopause, a strong decrease in ovarian hormone production occurs. Given the severity of the hormonal changes at these stages, developing effective treatments against sexual dysfunction for premenopausal and postmenopausal women is particularly challenging. Estradiol and testosterone treatments have shown promising results, however, these therapeutic strategies still present the risk of side effects such as endometrial cancer, breast cancer, cardiovascular disease, hirsutisms, and acne.

The present inventors surprisingly found that pharmaceutical compositions comprising the four plant extracts above described at particular weight ratios are also effective and safe in the treatment of sexual dysfunction in women undergoing strong hormonal shifts.

Hence, in a particular embodiment, the combination or the pharmaceutical composition of the invention is for use in the treatment and/or prevention of female sexual dysfunction in perimenopausal or postmenopausal women.

Non-therapeutic uses of the combination and pharmaceutical composition of the invention for improving female sexual function are also considered to form part of the present invention. Therefore, the invention also provides the use of the combination of the first aspect or the pharmaceutical composition of the second aspect for improving female sexual function. In a particular embodiment, the use is for improving female sexual function caused by a hormonal imbalance condition. In a particular embodiment, the hormonal imbalance condition is selected from the group consisting of pregnancy, postpartum, breastfeeding, hormonal contraception, perimenopause, postmenopause and combinations thereof. In particular, the hormonal imbalance condition is perimenopause or postmenopause.

In addition, this combination can be thought as sexual stimulant not for pathological conditions but for healthy individuals interested in improving their sexual function.

The high efficacy of the composition of the invention allows for a simple administration regime in which only one tablet a day comprising 600 mg of *Trigonella foenum graecum* is enough to produce significant results in female sexual function.

In a particular embodiment of the third aspect, the treatment comprises administering to a female mammal a dosage unit of the combination or the pharmaceutical composition comprising from 300 mg to 900 mg of *Trigonella foenum graecum* extract, from 75 to 225 mg of *Turnera diffusa* extract, and from 25 to 75 mg of *Ginkgo biloba* extract per day. In a more particular embodiment, the treatment comprises administering to a female mammal a dosage unit of the combination or the pharmaceutical composition comprising 600 mg of *Trigonella foenum graecum* extract, 150 mg of *Turnera diffusa* extract, and 50 mg of *Ginkgo biloba* extract per day.

In a particular embodiment of the third aspect, the treatment comprises administering to a female mammal two dosage units of the combination or the pharmaceutical composition comprising from 150 mg to 450 mg of *Trigonella foenum graecum* extract, from 30 to 120 mg of *Turnera diffusa* extract, and from 10 to 40 mg of *Ginkgo biloba* extract per day. In a more particular embodiment, the treatment comprises administering to a female mammal two dosage units of the composition comprising 300 mg of *Trigonella foenum graecum* extract, 75 mg of *Turnera diffusa* extract, and 25 mg of *Ginkgo biloba* extract per day.

In a particular embodiment of the third aspect, the treatment comprises administering to a female mammal a dosage unit of the combination or the pharmaceutical composition comprising from 300 mg to 900 mg of *Trigonella foenum graecum* extract, from 75 to 225 mg of *Turnera diffusa* extract, from 25 to 75 mg of *Ginkgo biloba* extract, and from 20 to 60 of *Tribulus terrestris* extract per day. In a more particular embodiment, the treatment comprises administering to a female mammal a dosage unit of the combination or the pharmaceutical composition comprising 600 mg of *Trigonella foenum graecum* extract, 150 mg of *Turnera diffusa* extract, 50 mg of *Ginkgo biloba* extract, and 40 mg of *Tribulus terrestris* extract per day.

In a particular embodiment of the third aspect, the treatment comprises administering to a female mammal two dosage units of the combination or the pharmaceutical composition comprising from 150 mg to 450 mg of *Trigonella foenum graecum* extract, from 30 to 120 mg of *Turnera diffusa* extract, from 10 to 40 mg of *Ginkgo biloba* extract, and from 10 to 30 of *Tribulus terrestris* extract per day. In a more particular embodiment, the treatment comprises administering to a female mammal a dosage unit of the combination or the pharmaceutical composition comprising 300 mg of *Trigonella foenum graecum* extract, 75 mg of *Turnera diffusa* extract, 25 mg of *Ginkgo biloba* extract, and 20 mg of *Tribulus terrestris* extract per day.

In a particular embodiment of the third aspect, the combination or the pharmaceutical composition is for use by oral administration.

According to the desired duration and effectiveness of the treatment, the combination or the pharmaceutical compositions of the invention may be administered once or several times, also intermittently, for example on a daily basis for several days, weeks or months and in different dosages. Particularly, the compositions of the invention are administered daily. More particularly, the treatment plan comprises administering one or two dosage units per day during at least 9 weeks.

The combination or the pharmaceutical composition of the invention can be prepared by conventional methods known to those skilled in the art.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Methods

In an observational, prospective, non-controlled study scheduled for 30 participants aged 45-65 with a < 25.83 FSFI (Female Function Sexual Index), this number being considered as the threshold for low sexual desire. All women were included at routine clinical visits and were treated with 2 tablets, one every 12 hours, daily for 2 months (9+-1 weeks). The concentration of active ingredients for every 2 tablets is the following:
- Dry *Trigonella foenum-grecum* seed extract: 600 mg (saponins: 300 mg) (stabilizer: microcrystaline cellulose).
- Dry Damiana (*Turnera diffusa* wild) leaf extract (4:1):150 mg (stabilizer: hydroxy propyl cellulose) (Geographical origin: North America).
- Dry Ginkgo (*Ginkgo biloba*) leaf extract: 50 mg (ginkgoflavonoids: 12.50 mg).
- Dry Tribulus (*Tribulus terrestris*) fruit extract: 40 mg (saponins: 36mg) (Geographical origin: Asia).
- Vitamin B3: 32 mg (anti-agglomerant: silicon dioxide; stabilizer: magnesium stearate).
- Vitamin B2: 2.8 mg.
- Vitamin B1: 2.2 mg.
- Vitamin B6: 2 mg.
- Selenium: 50 µg (110 µg of sodium selenite).

Although the geographical origin of some extracts has been indicated, other extracts with the same or similar composition can be used.

The coverage of the tablets comprised stabilizers (hydroxy propyl methyl cellulose and microcrystalline cellulose), fragrance, anti-agglomerants (stearic acid and potassium and aluminum silicate), colorants (titanium dioxide and red iron oxide, in particular Candurin® Gold Lustre).

The women's mean age was 53.9 (45-65). Demographic data are described in Table 1.

**Table 1 .Demographic characteristics (n=29)**

| | |
|---|---|
| Age (mean SD) | 54.69 (6.29) |
| Caucasian race | 100% |
| Mean weight (SD) | 63.33 (8.81) |
| Mean size (SD) | 159.40 (7.06) |
| Studies (n, %) | |
| Primary | 11 (39.2%) |
| Medium | 8 (28.5%) |
| Superior | 9 (32.3%) |
| Missing | 1 |
| Gynecological characteristics | |
| Mean menarche age (SD) | 12.62 (1.5) |
| Mean menopause age (SD) | 49.42 (6.92) |
| Hysterectomy n% | 4 (13.8%) |
| No hysterectomy n% | 25 (86.2%) |
| Mean deliveries (SD) | 1.03 (1.09) |

Sexual function was evaluated using the FSFI. The FSFI is a 19-item questionnaire divided into six domains: desire, excitement, lubrication, orgasm, satisfaction, and pain (c.f. Rosen R et al., "The female sexual function index (FSFI): a multidimensional self-report instrument for the assessment of female sexual function" 2000, J Sex Marital Ther, vol. 26, pp. 191-208). The differentiation threshold for sexual dysfunction was established at <25.83 (21). Score system: the individual score was achieved and then added to other scores from the same domain, multiplied by the relevant factor. The total scale was achieved by adding the scores obtained in the 6 domains. A 0 score in a given domain indicated no sexual activity in the last month. This tool provides with optimal psychometric properties for each of the 6 domains, it is easy to use, and it has been demonstrated to be able to discriminate between clinical populations (women with sexual dysfunction) and non-clinical populations (without sexual disorders).

The study was based on a selection stage in which, apart from the FSFI index, other inclusion criteria were also determined. This stage was followed by a 9-week treatment stage. The protocol was approved by the ethics committee our center (Instituto Palacios, Salud y Medicina de la Mujer, Madrid) belongs to.

The main inclusion criteria were:
- Women aged ≥ 45 and ≤ 65.
- Women with a stable partner, living together for at least 15 days a month and being sexually available.
- Sexual dysfunction established at < 25.83 FSFI scores
- Integrity of the vaginal mucosa (without lesions or bleeding).
- Women willing to and capable of understanding and signing an informed consent after receiving an explanation on the nature of the whole study.
- Consenting to participate in the study and signing the Informed Consent form.
- No desire for pregnancy in the next 3 months.

### Exclusion Criteria

- Non-compliance with the requirements above
- Pregnant women or with suspected pregnancy.
- Within 3 months following delivery or abortion.
- Breastfeeding women.
- Women with severe pain in sexual relationships.
- Non-diagnosed abnormal genital bleeding or presence of vaginal lesion.
- Women with symptoms of vaginal infection or signs of any other genital infection.
- Women allergic or with hypersensitivity to the components of the study treatment.
- Severe psychiatric disorder.
- Use of any hormonal treatment with estrogens, progestogens, or estrogens and progestogens in the 3 treatment months.
- Use of any other drug or experimental device within 30 days prior to selection.
- Any condition preventing the patient from participating in the study, at the researcher's discretion.

All participants received a written consent form and signed it before any procedure related to the study was carried out. Participants had a medical history, a medical examination, and a comprehensive gynecological check-up. A gynecological examination was performed to assess the aspect of the mucosa and the tolerance to medication on day 1 and on week 9.

### Laboratory Tests

Serum steroid levels of free testosterone and SHBG (Sex Hormone Blinding Globulin) were measured in the LABCO Laboratory. SHBG was measured with chemiluminescent immunoassay Immulite 2000 XPi (Siemens Healthcare Diagnostics, Eschborn, Germany) with an inter-assay coefficient of variation of 3.5% and 8.3% at low level, and 4.8% and 5.4% at high level, respectively. The estimation of serum free testosterone hormone levels was carried out using the ELISA technique. The DiaMetra Italy kit (DKO-015) was used to determine free testosterone hormone concentration in human serum according to the manufacturer's instructions.

### Analysis of Variables

The analyses were carried out both for the total of the sample in relation to FSFI, and for the sample of the determinations of free testosterone and SHBG. Hormonal determination was only performed in patients giving their consent to that. The characteristics of the analytical parameters (FSFI free testosterone and SHBG) in the two visits (pre/post), the absolute change between visits (difference post-pre), and the relative change between visits ([post-pre]/pre x 100%) were described.

### Statistical Methodology

A descriptive analysis of the variables included in the study was carried out. Central tendency and dispersion measures were presented, that is, mean, standard deviation (SD), median, percentiles 25 and 75 (P25 and P75, respectively), and minimum and maximum (min. and max., respectively) of quantitative variables.
Absolute (n) and relative (%) frequency distributions of qualitative variables were presented. Unavailable data were not allocated. They were just described as lost data.

To analyze the evolution/change throughout the study, parametric tests for continuous variables (Student's T test for paired samples) and/or non-parametric tests (Wilcoxon) were used.

The hypothesis testing was bilateral in all cases, with a significance level of p < 0.05. Analyses were carried out using the SAS statistical software, version 9.4.

### Results

The sample to be analyzed was made up of 29 patients who completed the study. One patient withdrew from the study as she had to travel. Of the 29 patients, 21 consented to blood tests for hormonal determination prior to and upon completion of the study.

Demographic data from patients are shown in Table 1. Mean age was 54.69. All women were Caucasian and from different educational and economic backgrounds. In this study group, all women were considered as healthy upon clinical history, exploration, and recent blood and biochemistry tests less than 3 months old.

FSFI results are shown in Table 2, which features the mean of baseline values and values after two months, with their standard deviations, of each domain, as well as global determination. As demonstrated by the results after treatment, values were significant for all domains except for dyspareunia. The domain presenting the highest increase was desire, with p=0.0004. The global domain increased 4.15 (6.14) points on average, with a statistical significance of p=0.0011.

**Table 2. Mean of baseline values and values after two months of treatment. Absolute change and statistical significance of the different FSFI domains**

| | Mean at baseline | Mean at 2 months | Absolute change (mean) | SS |
|---|---|---|---|---|
| Desire | 2.40 (0.96) | 3.33 (1.29) | 0.93 (1.25) | p=0.0004 |
| Excitement | 3.04 (0.96) | 3.63 (1.44) | 0.59 (1.25) | p=0.0166 |
| Lubrication | 3.70 (0.98) | 4.50 (1.34) | 0.80 (1.34) | p=0.0034 |
| Orgasm | 3.57 (1.14) | 4.23 (1.43) | 0.66 (1.30) | p=0.0106 |
| Sexual satisfaction | 4.01 (0.99) | 4.62 (1.22) | 0.61 (1.45) | p=0.0321 |
| Dyspareunia | 4.19 (1.06) | 4.76 (1.40) | 0.57 (1.55) | p=0.0602 |
| Global | 20.42 (4.23) | 25.08 (6.62) | 4.15(6.14) | p=0.0011 |

Regarding the number of patients whose FSFI increased beyond 26, that is, those who initially had sexual dysfunction scores and eventually reached normality scores, there were 18 of them (Table 3), which accounts for 62%. 89.9 % of patients experienced an increase in their FSFI following treatment.

**Table 3. Number of patients who ceased to have sexual dysfunction based on their FSFI score.**

| | N° | % |
|---|---|---|
| Patients >26 on FSFI score | 18 | 62 |
| Patients with increased score | 26 | 89,6 |
| Patients with decreased score | 3 | 10,4 |

As far as free testosterone data are concerned, Figure 1A shows the numbers at visit 1 (pre) and visit 2 (post), as well as the change between both visits. With a sample of 21 patients with available data, statistically significant differences were noted in testosterone values between visit 1 (pre) and visit 2 (post). Testosterone mean change between visits (post-pre) was an increase of 0.09 (SD 0.17) units (p=0.0386); the mean percent change as compared to baseline was 79.3% (p=0.0214).

Finally, SHBG data at visit 1 (pre) and visit 2 (post), as well as changes between both visits, are shown in Figure 1B. With a sample of 21 patients with available data, statistically significant differences were noted in SHBG values between visit 1 (pre) and visit 2 (post). Mean SHBG change between visits (post-pre) was a decrease of -12.05 (SD 11.48) units (p=0.0001); the mean percent change as compared to baseline was -13.17% (p<0.0001). 95.2% of patients assessed experienced a decrease in SHBG values (only 1 patient had increased SHBG values).

In summary, the results of this study show that the composition comprising the combination of extracts from *Trigonella foenum graecum* component, *Turnera Diffusa, Tribulus Terrestris,* and *Ginkgo* is safe and efficient for improving desire, excitement, lubrication, orgasm, and sexual satisfaction in women with sexual dysfunction. The results from this study made us think the composition under study may have a potential double effect, improving sexual desire through an increase in free testosterone, but also improving excitement by means of a vaginal vasodilating effect.

### Citation List

Davis S.R. et al., "Efficacy and safety of testosterone in the management of hypoactive sexual desire disorder in postmenopausal women", 2012, J Sex Med, vol. 9(4), pp. 1134-48.
Simon J.A. et al., "Efficacy and safety of flibanserin in postmenopausal women with hypoactive sexual desire disorder: results of the SNOWDROP trial" 2014, Menopause, vol. 21(6), pp. 633-40.
Aftab A. et al., "Flibanserin and its discontents" 2017, Arch Womens Ment Health, vol. 20(2), pp. 243-247.
Rosen R. et al., "The female sexual function index (FSFI): a multidimensional self-report instrument for the assessment of female sexual function" 2000, J Sex Marital Ther, vol. 26, pp. 191-208.

## Claims

1. A combination comprising extracts of *Trigonella foenum graecum, Turnera diffusa,* and *Ginkgo biloba* at a weight ratio from 6:1:1 to 24:6:1.

2. The combination according to claim 1, wherein the weight ratio between the extracts of *Trigonella foenum graecum, Turnera diffusa,* and *Ginkgo biloba* is 12:3:1.

3. The combination according to any of claims 1-2, further comprising an extract of *Tribulus terrestris.*

4. The combination according to claim 3, wherein the weight ratio between the extracts of *Trigonella foenum graecum, Turnera diffusa, Ginkgo biloba,* and *Tribulus terrestris* is from 6:1:1:0.2 to 24:6:1:2.

5. The combination according to claim 4, wherein the weight ratio between the extracts of *Trigonella foenum graecum, Turnera diffusa, Ginkgo biloba,* and *Tribulus terrestris* is 12:3:1:0.8.

6. The combination according to any of claims 1-5, wherein the extract of *Ginkgo biloba* is encapsulated into phytosomes.

7. The combination according to any of claims 1-6, further comprising vitamins and/or selenium.

8. The combination according to claim 7, wherein the vitamins are selected from the group consisting of vitamin B1, vitamin B2, vitamin B3, vitamin B6, and combinations thereof.

9. A pharmaceutical composition comprising an effective amount of the combination as defined in any of claims 1-8, together with one or more appropriate pharmaceutically acceptable excipients and/or carriers.

10. The combination as defined in any of claims 1-8, or the pharmaceutical composition as defined in claim 9, for use as a medicament.

11. The combination or the pharmaceutical composition for use according to claim 10, which is for use in the treatment and/or prevention of female sexual dysfunction.

12. The combination or the pharmaceutical composition for use according to claim 11, wherein the female sexual dysfunction is caused by a hormonal imbalance condition.

13. The combination or the pharmaceutical composition for use according to claim 12, wherein the hormonal imbalance condition is selected from the group consisting of pregnancy, postpartum, breastfeeding, hormonal contraception, perimenopause, and postmenopause.

14. The combination or the pharmaceutical composition for use according to claim 13, wherein the hormonal imbalance condition is perimenopause or postmenopause.

15. The combination or the pharmaceutical composition for use according to any of claims 10-14, wherein the treatment comprises administering to a female mammal a dosage unit of the combination or the composition comprising from 300 mg to 900 mg of *Trigonella foenum graecum* extract, from 75 to 225 mg of *Turnera diffusa* extract, and from 25 to 75 mg of *Ginkgo biloba* extract per day.
